# EUROPEAN PATENT SPECIFICATION

(11) **EP 3 828 539 B1**
(45) Date of publication and mention of the grant of the patent: **03.09.2025**
(21) Application number: 19841936.8
(22) Date of filing: 19.07.2019
(51) Int. Cl.: G01N 33/483, G01N 25/20, G01N 25/48, G01N 33/487, G01N 33/49

(54) **METHOD FOR PROVIDING CANCER DIAGNOSIS INFORMATION USING THERMAL ANALYSIS METHOD AND PORTABLE CANCER DIAGNOSIS DEVICE USING THERMAL ANALYSIS METHOD**
VERFAHREN ZUR BEREITSTELLUNG VON KREBSDIAGNOSEINFORMATION UNTER VERWENDUNG EINES THERMOANALYSEVERFAHRENS UND TRAGBARE KREBSDIAGNOSEVORRICHTUNG UNTER VERWENDUNG EINES THERMOANALYSEVERFAHRENS
PROCÉDÉ DESTINÉ À FOURNIR DES INFORMATIONS DE DIAGNOSTIC DU CANCER À L'AIDE D'UN PROCÉDÉ D'ANALYSE THERMIQUE ET DISPOSITIF DE DIAGNOSTIC DU CANCER PORTABLE À L'AIDE D'UN PROCÉDÉ D'ANALYSE THERMIQUE

(30) Priority: 25.07.2018 KR 20180086356; 17.06.2019 KR 20190071239; 12.07.2019 KR 20190084120
(43) Date of publication of application: 02.06.2021
(73) Proprietor: Industry-Academic Cooperation Foundation, Kunsan National University, Gunsan-si, Jeollabuk-do 54150 (KR)
(72) Inventor: JOO, Ik Su, Daegu 42779 (KR); SUN, Ho Jung, Gunsan-si Jeollabuk-do 54101 (KR); YOO, Seong Hee, Gunsan-si Jeollabuk-do 54155 (KR); LEE, Sang Bum, Gunsan-si Jeollabuk-do 54149 (KR)
(74) Representative: V.O.
(86) International application number: PCT/KR2019/008981
(87) International publication number: WO 2020/022714

(56) References cited:
- WO-A1-2017/122174
- US-A1- 2011 301 860
- TODINOVA, SVETLA: "Unusual thermal transition in the serum calorimetric profile of a patient diagnosed with multiple myeloma with secretion of monoclonal k free light chains: a case report", CANCER RESEARCH FRONTIER, vol. 2, no. 3, 2016, pages 416 - 426, XP055679609
- KIKALISHVILI, L.: "Thermal stability of blood plasma proteins of breast cancer patients, DSC study", JOURNAL OF THERMAL ANALYSIS AND CALORIMETRY, vol. 120, 2015, pages 501 - 505, XP035476326, DOI: 10.1007/s10973-015-4426-2
- KEDRA-KROLIK, KAROLINA: "Blood serum calorimetry indicates the chemotherapeutic efficacy in lung cancer treatment", SCIENTIFIC REPORTS, vol. 7, 2017, pages 1 - 5, XP055679611

## Description

### [Technical Field]

The present disclosure relates to a method for providing cancer diagnosis information using a thermal analysis method and a portable cancer diagnosis device using a thermal analysis method.

### [Background Art]

Cancer is one of the most universal cause of death globally. About 10 million new cases occur annually, and it is the third cause of death accounting for about 12% of all deaths.

The existing cancer diagnosis methods such as magnetic resonance imaging, endoscopy, biopsy, chemical test, etc. are disadvantageous in that they may be harmful to the human body, require long time for diagnosis, and are costly.

Cancer cells have higher temperatures than nearby normal cells because they proliferate faster than normal cells and exhibit thermal reactions. Methods for diagnosing cancer using these characteristics of the cancer cells are disclosed in a number of patents and literatures.

However, cancer diagnosis based on simple temperature measurement has problems that errors may occur because systematic data are insufficient and accurate measurement is difficult due to external factors such as the outside temperature, the age of a subject, the body temperature of the subject, the presence of trauma, etc.

A case report on an unusual thermal transition in a serum calorimetric profile of a patent diagnosed with multiple myeloma is provided by Todinova et al. (Cancer Research Frontiers, 2016 Sept; 2(3): 416-426). Here differential scanning calorimetry (DSC) is used to study thermal transitions in blood serum of a multiple myeloma patient.

WO 2017/122174 A1 discloses differential scanning calorimeter useful for e.g. detecting thermostable variants of proteins or metabolites in biological sample and diagnosing pathogenic infection comprises a furnace, a heater, and a reference channel and test channel.

### [Disclosure]

### [Technical Problem]

The present disclosure is directed to providing a method for providing cancer diagnosis information using a thermal analysis method according to various exemplary embodiments of the present disclosure.

The present disclosure is also directed to providing a portable cancer diagnosis device using a thermal analysis method according to various aspects and exemplary embodiments of the present disclosure.

### [Technical Solution]

An aspect of the present disclosure relates to a method for providing cancer diagnosis information, which includes: a step of obtaining a biological sample; a step of acquiring heat flux data of the biological sample in a temperature range of 37-47 °C; and a step of providing cancer diagnosis information based on the heat flux data.

According to the method for providing cancer diagnosis information of the present disclosure, the biological sample is one or more selected from a group consisting of cellular tissue, whole blood, urine, stool, sputum, saliva, tissue, cell and in-vitro cell culture.

According to another exemplary embodiment of the present disclosure, the heat flux data may be one or more selected from a group consisting of thermochemical reaction onset temperature, heat flow change, calorie change, power compensation and compensation temperature.

According to another exemplary embodiment of the present disclosure, the step of acquiring the heat flux data may be performed by differential scanning calorimetry (DSC), isothermal titration calorimetry (ITC) or differential thermal analysis (DTA).

According to another exemplary embodiment of the present disclosure, the step of acquiring the heat flux data may be performed by differential scanning calorimetry and may include a step of heating the biological sample to 37-47 °C at a rate of 1-10 °C/min.

According to another exemplary embodiment of the present disclosure, the step of acquiring the heat flux data may further include a stabilization step maintaining the temperature of the biological sample at 25-30 °C for 1-60 minutes before reaching the temperature at which the heat flux data is acquired.

According to another exemplary embodiment of the present disclosure, the step of acquiring the heat flux data may be performed by differential scanning calorimetry, and, in the step of providing cancer diagnosis information based on the heat flux data, diagnostic information about the presence of cancer may be provided based on the thermochemical reaction onset temperature of the biological sample.

Another aspect of the present disclosure relates to a portable cancer diagnosis device using a thermal analysis method, which includes: a sample reception unit to which a biological sample is introduced; a heating unit which heats the sample reception unit; a first temperature sensor which measures the temperature of the sample reception unit; a power supply unit which supplies power to the heating unit; and a control unit which determines the presence of cancer based on the temperature change data of the sample reception unit with time measured by the first temperature sensor.

Another aspect of the present disclosure relates to a portable cancer diagnosis device using a thermal analysis method, which includes: a sample reception unit to which a biological sample is introduced; a heating unit which heats the sample reception unit; a first temperature sensor which measures the temperature of the sample reception unit; and an interface unit which transmits the temperature change data of the sample reception unit with time measured by the first temperature sensor to a control unit of an electronic device and supplies power from the electronic device to the heating unit, wherein the control unit of the electronic device determines the presence of cancer based on the temperature change data with time transmitted from the interface unit.

According to an exemplary embodiment, the control unit may split the temperature of the temperature change data of the sample reception unit with time into a time zone A below a first predetermined temperature, a
time zone B between the first predetermined temperature and a second predetermined temperature, and a time zone C above the second predetermined temperature, and may determine the presence of cancer by measuring the time spent in the time zone B.

According to another exemplary embodiment, the control unit may determine the presence of cancer by measuring the temperature of the sample reception unit for a predetermined time from the temperature change data of the sample reception unit with time.

According to another exemplary embodiment, the control unit may represent the temperature change data of the sample reception unit with time on a graph with time on the x-axis and temperature on the y-axis, and may determine the presence
of cancer by analyzing the rough shape of a graph obtained by differentiating the graph with respect to time.

According to another exemplary embodiment, the control unit may represent the temperature change data of the sample reception unit with time on a graph with time on the x-axis and temperature on the y-axis, may split the time zone in the graph into a time zone A corresponding to below a first predetermined temperature, a time zone B corresponding to between the first predetermined temperature and a second predetermined temperature, and a time zone C corresponding to above the second predetermined temperature, and may determine the presence of cancer by analyzing the rough shape of a graph obtained by differentiating the graph with respect to time for the time zone B.

According to another exemplary embodiment, the portable cancer diagnosis device using a thermal analysis method may control the temperature of the heating unit by applying a fixed power corresponding to a predetermined maximum target temperature to the heating unit.

According to another exemplary embodiment, the portable cancer diagnosis device using a thermal analysis method may control the heating unit to be heated at a predetermined heating rate by raising the power suppled to the heating unit at a predetermined ratio with time.

According to another exemplary embodiment, the portable cancer diagnosis device using a thermal analysis method may further include a second temperature sensor which measures the temperature of the heating unit, and the control unit may control the heating unit to be heated at a predetermined heating rate using temperature data measured by the second temperature sensor.

According to another exemplary embodiment, the control unit may determine the presence of cancer by measuring the power supplied to the heating unit with time.

For the portable cancer diagnosis device using a thermal analysis method, the biological sample is one or more selected from a group consisting of cellular tissue, blood and body fluid.

### [Advantageous Effects]

A method for providing cancer diagnosis information using a thermal analysis method according to the present disclosure is capable of diagnosing the type, progression, metastasis, etc. of cancer using the thermochemical reaction onset temperature, calorie change, etc. obtained by analyzing the heat flux to and from a biological sample. Since the diagnosis is possible even with cellular tissue or blood, the type, progression, metastasis, etc. of cancer can be diagnosed simply in short time without the existing complicate procedures such as tissue culture, etc.

In addition, a portable cancer diagnosis device using a thermal analysis method of the present disclosure is capable of diagnosing the presence of cancer accurately and easily using the temperature function data, etc. depending on time measured by heating a biological sample. Since cancer diagnosis is possible even with cellular tissue or blood in a manner similar to self-monitoring of blood glucose, the diagnosis can be made in short time without the existing complicate procedures such as tissue culture, etc. **In** addition, since self-diagnosis is possible at low cost without limitation in space, the effect of primary prevention of cancer such as prevention, early detection, monitoring, etc. is provided and health self-care becomes possible.

### [Brief Description of Drawings]

FIG. 1 shows a result of differential scanning calorimetry for water according to an exemplary embodiment of the present disclosure.
FIG. 2 shows a result of differential scanning calorimetry for a medium according to an exemplary embodiment of the present disclosure.
FIG. 3A and FIG. 3B show a result of differential scanning calorimetry for normal cells according to an exemplary embodiment of the present disclosure.
FIG. 4A shows a result of differential scanning calorimetry for a mixture of cancer cells and a medium according to an exemplary embodiment of the present disclosure, and FIGS. 4B and 4C compare results of differential scanning calorimetry for normal cells and cancer cells according to an exemplary embodiment of the present disclosure. FIG. 4D shows a result of additional test for cancer cells according to an exemplary embodiment of the present disclosure.
FIG. 5 shows a result of repeating differential scanning calorimetry 3 times for the same cancer cells according to an exemplary embodiment of the present disclosure.
FIG. 6 schematically shows a portable cancer diagnosis device using a thermal analysis method used independently according to an exemplary embodiment of the present disclosure.
FIG. 7 schematically shows a portable cancer diagnosis device using a thermal analysis method used together with an electronic device according to an exemplary embodiment of the present disclosure.
FIG. 8 shows a graph describing the concept of a cancer diagnosis method using a portable cancer diagnosis device of the present disclosure.
FIG. 9 shows a graph describing the concept of a cancer diagnosis method using a portable cancer diagnosis device with the heating rate of a heating unit controlled according to an exemplary embodiment of the present disclosure.

### [Best Mode]

Hereinafter, the present disclosure is described in detail.

The existing cancer diagnosis methods such as magnetic resonance imaging, endoscopy, biopsy, chemical test, etc. are disadvantageous in that they may be harmful to the human body, require long time for diagnosis, and are costly.

Since a method for providing cancer diagnosis information using a thermal analysis method of the present disclosure performs diagnosis based on thermal analysis of a biological sample such as blood, cellular tissue, etc., the complicated procedure of culturing cellular tissue is omitted. In addition, the method is not harmful to the human body, does not require high cost, and is very effective with a very short measurement time of several to tens of minutes.

An aspect of the present disclosure provides a method for providing cancer diagnosis information, which includes: a step of obtaining a biological sample; a step of acquiring heat flux data of the biological sample in a temperature range of 37-47 °C; and a step of providing cancer diagnosis information based on the heat flux data.

Cancer cells have higher temperatures than nearby normal cells because they proliferate faster than normal cells and exhibit thermal reactions. Methods for diagnosing cancer using these characteristics of the cancer cells are disclosed in a number of patents and literatures.

However, cancer diagnosis based on simple temperature measurement has problems that errors may occur because systematic data are insufficient and accurate measurement is difficult due to external factors such as the outside temperature, the age of a subject, the body temperature of the subject, the presence of trauma, etc.

In contrast, according to the present disclosure, accurate diagnosis is possible without errors resulting from individual and external factors because the information for diagnosis is provided through measurement of the temperature of endothermal and exothermal reactions, heat flow change, etc. of the biological sample.

Vigorously growing cancer cells exhibit thermochemical reactions at 38-45 °C and absorb the supplied heat. Endothermal reactions occur when the cancer cells are activated, denatured, necrotized or killed. Therefore, cancer diagnosis is possible by analyzing thermal behavior after applying heat to cells or blood.

In addition, whereas the previously reported methods based on temperature analysis are performed at relatively high temperatures of 60 °C or higher, the present disclosure is advantageous in that cancer diagnosis information can be provided by detecting heat flux at low temperatures of 38-45 °C.

According to the method for providing cancer diagnosis information of the present disclosure, the biological sample is one or more selected from a group consisting of cellular tissue, whole blood, urine, stool, sputum, saliva, tissue, cell, in-vitro cell culture. According to the method for providing cancer diagnosis information of the
present disclosure, the result can be obtained simply without any pretreatment by monitoring thermal specificity of cells in a medium.

According to another exemplary embodiment of the present disclosure, the heat flux data may be one or more selected from a group consisting of thermochemical reaction onset temperature, heat flow change, calorie change, power compensation and compensation temperature.

According to another exemplary embodiment of the present disclosure, the step of acquiring the heat flux data may be performed by differential scanning calorimetry (DSC), isothermal titration calorimetry (ITC) or differential thermal analysis (DTA). However, any method capable of detecting heat flux to and from a biological sample, thermal (power) compensation, etc. may be used without being limited thereto.

Differential scanning calorimetry (DSC) is one of thermal analysis methods improved from differential thermal analysis (DTA). While heating or cooling a sample and a reference material at a constant rate under the same condition, the difference in calorie applied electrically such that the temperature of the two materials is equal is recorded on the ordinate, and the temperature (or time) is recorded on the abscissa. Although DTA is related with thermal conduction in the sample, quantitative measurement of calorie is difficult. Contrastingly, in DSC, because the sample and the reference material are heated with different devices, temperature difference occurs when they are heated or cooled at a constant rate, and energy is supplied to compensate for the difference. Through this measurement, the specific heat of the sample, the temperature of first-order phase transition, etc. may be determined. It is widely used in food industry for the study of starch gelatinization and aging, sol-gel transition of polysaccharide gel, solidification of egg white by heating, denaturation of other proteins, determination of solid fat index, determination of the crystallinity of cocoa butter, interaction of proteins, sugars, etc. with water, presence and state of water in food, quality evaluation of alcoholic drinks or chocolate, etc. This method is characterized in that measurement is possible with a relatively small amount of sample and manipulation is easy and automated.

Isothermal titration calorimetry (ITC) is used for quantitative study of the interactions of biomolecules and the heat released or absorbed during the interactions of the biomolecules can be measured directly. Because heat flux to and from a biological sample can be detected by the differential scanning calorimetry and the isothermal titration calorimetry, they may be used in the method for providing cancer diagnosis information of the present disclosure.

For example, the type of cancer can be distinguished by measuring the thermochemical reaction onset temperature of the biological sample in a temperature range of 38-45 °C by differential scanning calorimetry, and the progression of cancer can be monitored from the DSC endothermic peak area data in the corresponding temperature range.

According to another exemplary embodiment of the present disclosure, the step of acquiring the heat flux data may include a step of heating the biological sample to 37-47 °C at a rate of 1-10 °C/min. If the heating rate is outside the range of 1-10 °C/min, it may be difficult to provide accurate cancer diagnosis information due to errors in the sensitivity and accuracy of the heat flux data.

According to another exemplary embodiment of the present disclosure, the step of acquiring the heat flux data may further include a step of maintaining the temperature of the biological sample at 25-30 °C for 1-60 minutes. When the step of maintaining the temperature of the biological sample at 25-30 °C for 1-60 minutes is included further, the stability and accuracy of the heat flux data may be improved.

But, in the step of maintaining the temperature of the biological sample at 25-30 °C for 1-60 minutes and the step of heating the biological sample to 37-47 °C at a rate of 1-10 °C/min, the temperature ranges, the time and the heating rate are not limited to those described above and may be controlled adequately depending on the type of the biological sample within ranges where the accuracy and reproducibility of achieved are maintained.

According to another exemplary embodiment of the present disclosure, the step of acquiring the heat flux data may be performed by differential scanning calorimetry and, in the step of providing cancer diagnosis information based on the heat flux data, diagnostic information about the presence of cancer may be provided based on the thermochemical reaction onset temperature of the biological sample. As described above, endothermal reactions occur at temperature ranges of 38-45 °C during differential scanning calorimetry of cancer cells, unlike normal cells. Diagnostic information about the presence of cancer may be provided depending on the presence of absence of thermochemical reactions using these characteristics of cancer cells.

According to another exemplary embodiment of the present disclosure, the cancer diagnosis information may include the type, progression and metastasis of cancer.

According to another exemplary embodiment of the present disclosure, the type of cancer may be one or more type of cancer selected from a group consisting of blood cancer, liver cancer, lung cancer, bladder cancer, stomach cancer, colorectal cancer, bile duct cancer, breast cancer, uterine cancer, colon cancer, ovarian cancer, pancreatic cancer, prostate cancer, bone cancer, skin cancer, laryngeal cancer, nasopharyngeal cancer, small intestine cancer, thyroid cancer, parathyroid cancer, urethral cancer, bronchial cancer, kidney cancer and bone marrow cancer.

The step of providing cancer diagnosis information may be developed as a statistical algorithm. The statistical algorithm may be one developed based on a database including the thermal analysis data of the biological sample, the thermochemical reaction onset temperature data depending on the type of cancer, the calorie data of endothermic peaks, etc. Accordingly, the type and progression of cancer can be monitored by applying the result of thermal analysis of unknown cellular tissue or blood to the statistical algorithm. The statistical algorithm may be applied individually to a program of a personal computer, an application of a mobile phone, etc.

Another aspect of the present disclosure provides a portable cancer diagnosis device using a thermal analysis method, which includes: a sample reception unit 10 to which a biological sample is introduced; a heating unit 20 which heats the sample reception unit 10; a first temperature sensor 30 which measures the temperature of the sample reception unit 10; a power supply unit 60 which supplies power to the heating unit 20; and a control unit 40 which determines the presence of cancer based on the temperature change data of the sample reception unit 10 with time measured by the first temperature sensor 30.

Another aspect of the present disclosure provides a portable cancer diagnosis device using a thermal analysis method, which includes: a sample reception unit 10 to which a biological sample is introduced; a heating unit 20 which heats the sample reception unit 10; a first temperature sensor 30 which measures the temperature of the sample reception unit 10; and an interface unit 50 which transmits the temperature change data of the sample reception unit 10 with time measured by the first temperature sensor 30 to a control unit of an electronic device and supplies power from the electronic device to the heating unit 20, wherein the control unit of the electronic device determines the presence of cancer based on the temperature change data with time transmitted from the interface unit.

Cancer diagnosis based on simple temperature measurement has problems that errors may occur because systematic data are insufficient and accurate measurement is difficult due to external factors such as the outside temperature, the age of a subject, the body temperature of the subject, the presence of trauma, etc.

**In** contrast, with the portable cancer diagnosis device using a thermal analysis method of the present disclosure, accurate diagnosis is possible without errors resulting from individual and external factors because the information for diagnosis is provided through measurement of the temperature of endothermal and exothermal reactions, heat flow change, etc. of the biological sample.

Vigorously growing cancer cells exhibit thermochemical reactions at 37-47 °C and absorb the supplied heat. Endothermal reactions occur when the cancer cells are activated, denatured, necrotized or killed. Therefore, cancer diagnosis is possible by analyzing thermal behavior after applying heat to cells or blood.

In addition, whereas the previously reported methods based on temperature analysis are performed at relatively high temperatures of 60 °C or higher, the present disclosure is advantageous in that cancer diagnosis information can be provided by detecting heat flux at low temperatures of 37-47 °C.

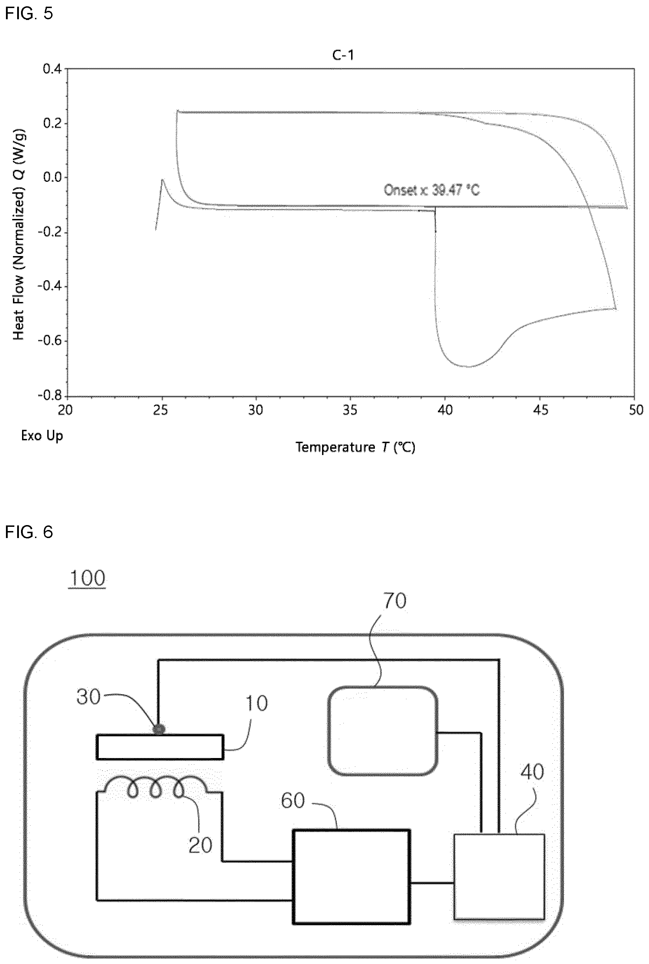

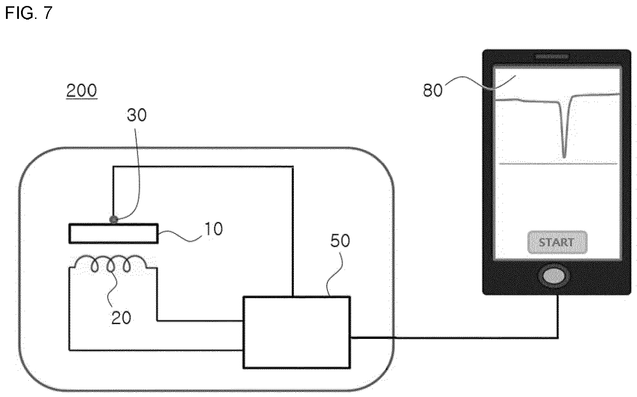

As described above, the portable cancer diagnosis device using a thermal analysis method of the present disclosure can be used either independently or together with an electronic device. FIG. 6 schematically shows an independent portable cancer diagnosis device 100 using a thermal analysis method used independently according to an exemplary embodiment of the present disclosure, and FIG. 7 schematically shows an attachable portable cancer diagnosis device 200 using a thermal analysis method used together with an electronic device according to an exemplary embodiment of the present disclosure.

The control unit 40 may split the temperature of the temperature change data of the sample reception unit 10 with time into a time zone A below a first predetermined temperature, a time zone B between the first predetermined temperature and a second predetermined temperature, and a time zone C above the second predetermined temperature, and may determine the presence of cancer by measuring the time spent in the time zone B.

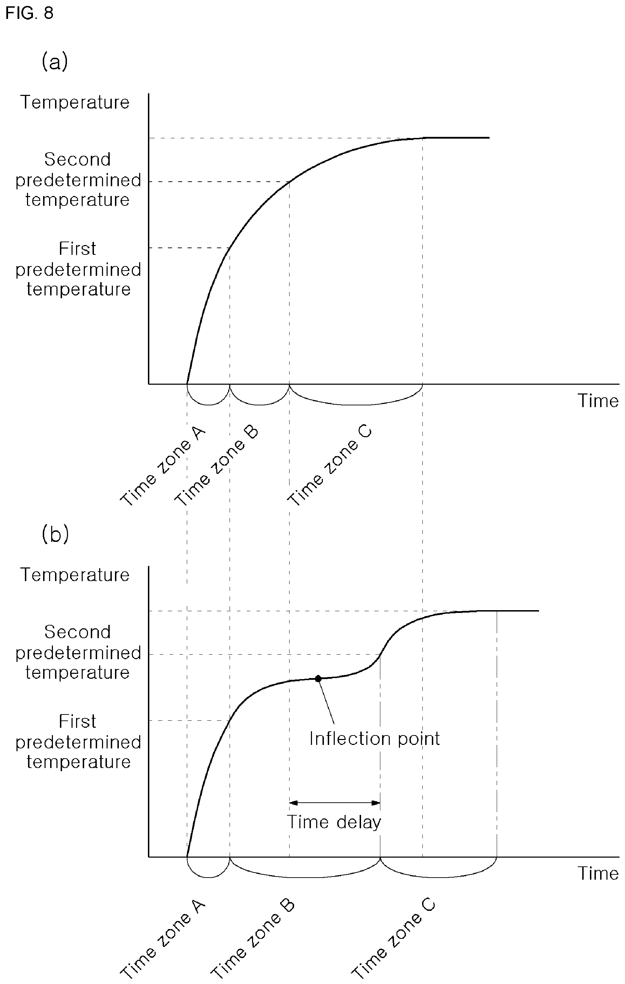

FIG. 8 shows a graph describing the concept of a cancer diagnosis method using a portable cancer diagnosis device of the present disclosure. FIG. 8a shows a temperature-time graph of normal cells for illustrating the concept of the cancer diagnosis method of the portable cancer diagnosis device of the present disclosure, and FIG. 8b shows a temperature-time graph of normal cells for cancer cells. Referring to FIG. 8a and 8b, the temperature-time graph of the cancer cells shows a characteristic inflection point when compared with the temperature-time graph of the normal cells, and time delay occurs in a specific temperature range when compared with the temperature-time graph of the normal cells. Accordingly, cancer may be diagnosed if significant difference occurs between the predetermined temperature change data of normal cells with time and the time required until the sample reception unit 10 reaches a predetermined temperature, measured by the portable cancer diagnosis device.

The first predetermined temperature is 34-38 °C and the second predetermined temperature **is** 42-47 °C. Specifically, the first predetermined temperature may be 35-37 °C and the second predetermined temperature may be 45-47 °C. The first predetermined temperature is the temperature at which the endothermic reaction of cancer cells begins, and the second predetermined temperature is the temperature at which the endothermic reaction is terminated. As described above, the temperature range between the first predetermined temperature and the second predetermined temperature is set as the time zone B. Accurate diagnosis becomes possible since only the data of the time zone B can be selected for the diagnosis after filtering out the data of other temperature ranges where thermochemical reaction does not occur and, thus, are useless in cancer diagnosis.

In addition, the control unit 40 may determine the presence of cancer by measuring the temperature of the sample reception unit 10 for a predetermined time from the temperature change data of the sample reception 10 unit with time. As described above, there exists a zone where temperature is increased between 37 and 47 °C due to endothermal reactions of cancer cells. Accordingly, by measuring the temperature of the sample reception unit 10 for a predetermined time, it is possible to diagnose cancer if the temperature is below the expected temperature. Specifically, the predetermined time may be set as the time immediately before the termination of thermochemical reactions of cancer cells, so that the delay of temperature increase can be measured distinctly. Accordingly, it may be set as the time until the temperature reaches 45-47 °C, which is the range where the thermochemical reactions of cancer cells are terminated.

The control unit 40 may represent the temperature change data of the sample reception unit 10 with time on a graph with time on the x-axis and temperature on the y-axis, and may determine the presence of cancer by analyzing the rough shape of a graph obtained by differentiating the graph with respect to time.
For cancer cells, there exists a range where the rate of change of temperature decreases rapidly due to endothermal reactions, unlike normal cells. Accordingly, the graph obtained by differentiating the graph with time on the x-axis and temperature on the y-axis with respect to time shows a characteristic rough shape including peaks the derivatives of which decrease rapidly and then increase rapidly. This enables the diagnosis of the presence of cancer in a sample. This is advantageous in that accurate diagnosis is possible even when the heating rate of the heating unit 20 cannot be controlled precisely.

The control unit 40 represents the temperature change data of the sample reception unit 10 with time on a graph with time on the x-axis and temperature on the y-axis, splits the time zone in the graph into a time zone A corresponding to below a first predetermined temperature, a time zone B corresponding to between the first predetermined temperature and a second predetermined temperature, and a time zone C corresponding to above the second predetermined temperature, and determines the presence of cancer by analyzing the rough shape of a graph obtained by differentiating the graph with respect to time for the time zone B.

The first predetermined temperature is 34-38 °C and the second predetermined temperature is 42-47 °C. Specifically, the first predetermined temperature is 35-37 °C and the second predetermined temperature is 45-47 °C. The first predetermined temperature is the temperature at which the endothermic reaction of cancer cells begins, and the second predetermined temperature is the temperature at which the endothermic reaction is terminated. As described above, the temperature range between the first predetermined temperature and the second predetermined temperature is set as the temperature zone B. Accurate diagnosis becomes possible since only the data of the temperature zone B can be selected for the diagnosis after filtering out the data of other temperature ranges where thermochemical reaction does not occur and, thus, are useless in cancer diagnosis.

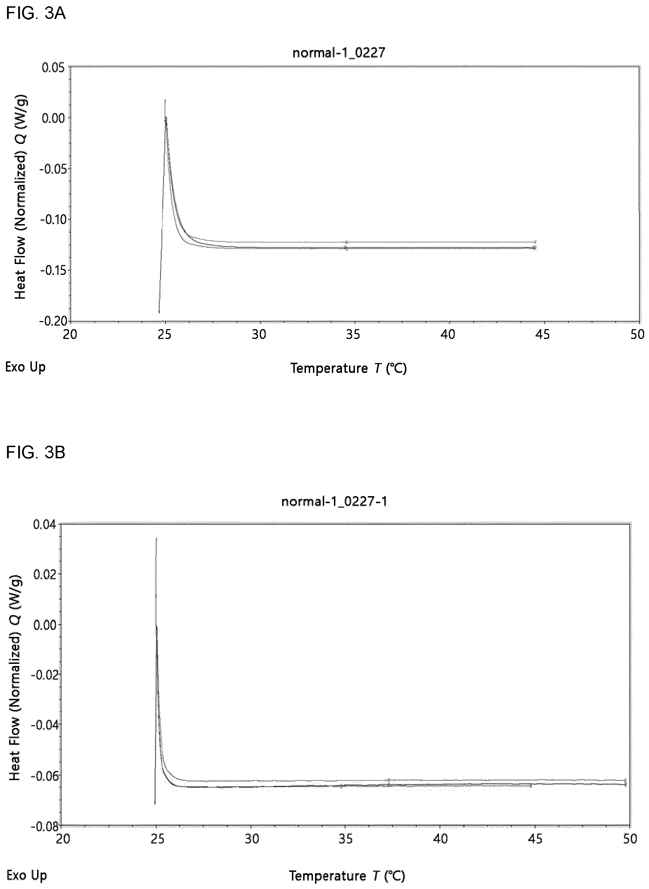

According to another exemplary embodiment, the portable cancer diagnosis device using a thermal analysis method may control the temperature of the heating unit 20 by applying a fixed power corresponding to a predetermined maximum target temperature to the heating unit 20. Specifically, the predetermined maximum target temperature may be 50 °C or higher for complete detection of the thermochemical reactions of cancer cells. FIGS. 3A and 3B show examples wherein the temperature of the heating unit 20 is increased by applying a fixed power corresponding to a maximum target temperature of 50 °C.

According to another exemplary embodiment, the portable cancer diagnosis device using a thermal analysis method may control the heating unit 20 to be heated at a predetermined heating rate by raising the power suppled to the heating unit 20 at a predetermined ratio with time.

According to another exemplary embodiment, the portable cancer diagnosis device using a thermal analysis method may further include a second temperature sensor which measures the temperature of the heating unit 20, and the control unit 40 may control the heating unit 20 to be heated at a predetermined heating rate using temperature data measured by the second temperature sensor.

Even when the second temperature sensor which measures the temperature of the heating unit 20 is not equipped, the heating rate can be controlled to some extent by increasing the power applied to the heating unit 20 with time at a predetermined ratio.

But, when the second temperature sensor which measures the temperature of the heating unit 20 is equipped additionally, the temperature can be controlled to increase linearly with time regardless of the amount of the biological sample or other conditions. That is to say, it becomes possible to measure the temperature of heating unit 20 and the sample reception unit 10 at the same time and to more precisely control the heating rate of the heating unit 20 corresponding to the temperature data. As a result, more accurate diagnosis becomes possible.

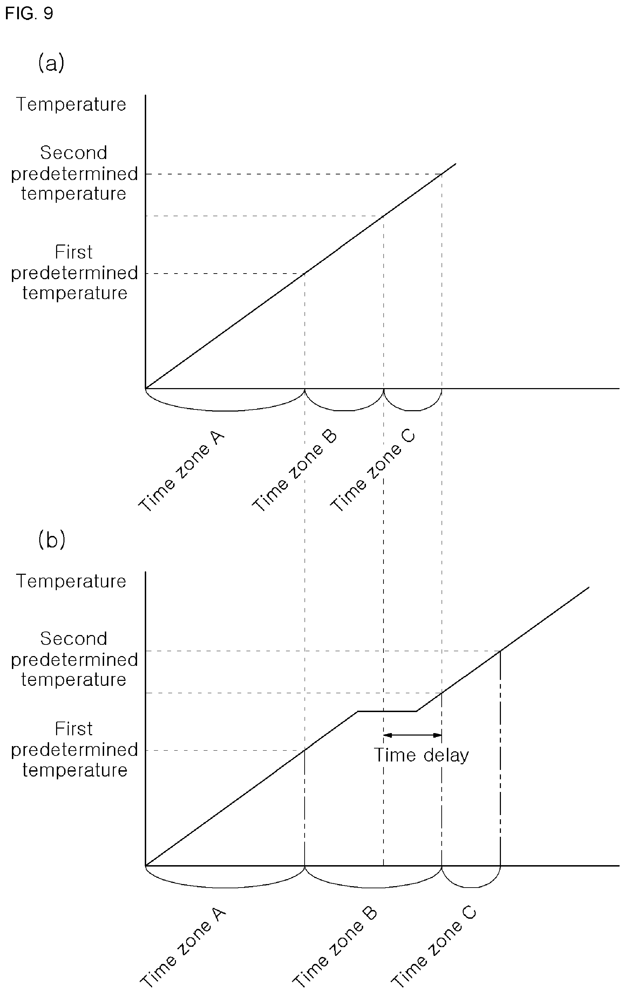

FIG. 9 shows a graph describing the concept of the cancer diagnosis method using a portable cancer diagnosis device with the heating rate of a heating unit controlled according to an exemplary embodiment of the present disclosure. FIG. 9a shows the temperature-time graph of the heating unit 20 with the heating rate controlled, and FIG. 9b shows the temperature-time graph of the sample reception unit 10 accommodating cancer cells heated by the heating unit 20. If the change of the temperature of the heating unit 20 with time is controlled linearly as shown in FIG. 9a, the range where the increase of temperature is delayed in the temperature-time graph of cancer cells can be detected clearly as shown in FIG. 9b and, therefore, the precision of diagnosis may be improved. By differentiating the graph with respect to time, the accuracy of diagnosis can be further improved.

The predetermined heating rate may be 0.1-10 °C/min, specifically 1-5 °C/min. If the heating rate is slower than 0.1 °C/min, a long time is required for measurement because the sample is heated very slowly. And, if the heating rate exceeds 10 °C/min, precise diagnosis may be difficult due to the excessively fast heating rate.

The control unit 40 may determine the presence of cancer by measuring the power supplied to the heating unit 20 with time. In order to determine the presence of cancer by measuring the power with time, the second temperature sensor described above may be necessary. As described above, when the second temperature sensor is equipped additionally, the temperature of the sample reception unit 10 may be increased at a constant heating rate regardless of the thermochemical reactions of cancer cells. Because the temperature of the sample reception unit and the heating unit 20 is measured by the first temperature sensor 30 and the second temperature sensor, respectively, the power supplied to the heating unit 20 may be controlled in real time such that the sample reception unit 10 is heated at a constant heating rate through feedback between the temperature sensors. Because the amount of supplied power is increased rapidly during the characteristic endothermal reactions of cancer cells in order to maintain the heating rate, it is possible to determine the presence of cancer by measuring the rapidly increased amount of supplied power. This is advantageous in that more accurate diagnosis is possible since the measurement method is not affected by the amount of the biological sample accommodated in the sample reception unit 10, onset temperature, etc.

When the portable cancer diagnosis device using a thermal analysis method is used as being attached to an electronic device, its operation can be controlled by an interface unit 50 connected to the electronic device without an additional control unit for operation of the device. The interface unit 50 may receive power supply and control signals from the electronic device. That is to say, the interface unit 50 may not only transmit the temperature change data with time measured by the first temperature sensor 30 to the control unit of the electronic device but also operate the heating unit 20 by transmitting the power supplied from the control unit of the electronic device to the heating unit 20. In addition, it may operate or stop the heating unit 20 based on the transmitted signal.

For this, the portable cancer diagnosis device using a thermal analysis method used as being attached to the electronic device may include an application or a program for operation of the electronic device in the control unit of the electronic device.

The biological sample for the portable cancer diagnosis device using a thermal analysis method may be one or more selected from a group consisting of cellular tissue, blood and body fluid, although not being limited thereto. The biological sample may include whole blood, serum, blood plasma, urine, stool, sputum, saliva, tissue, cell, cell extract, in-vitro cell culture, etc. According to the method for providing cancer diagnosis information of the present disclosure, the result can be obtained simply without any pretreatment by monitoring thermal specificity of cells in a medium.

Specifically, the sample container may be prepared from a material with high thermal conductivity for precise analysis of the thermal behavior of the biological sample.

The sample container may be prepared from a metal coated with a carbon-based material, and the metal may be a metal having superior thermal conductivity, which is one or more selected from gold, silver, copper and aluminum.

Since the carbon-based material such as graphite, graphene, carbon nanotube, etc. has superior thermal conductivity, improved thermal conductivity can be expected when it is coated on the outside of the container.

More specifically, the sample container may have a thermal conductivity of 100 W/m·K or higher. Since the presence of cancer is diagnosed based on the analysis of the precise thermal behavior of the sample, diagnosis with high reliability cannot be achieved when the thermal conductivity is below 100 W/m·K.

The heating unit 20 heats the sample reception unit 10 to which the biological sample has been introduced using the power supplied from the power supply unit 60, and heats the sample reception unit 10 by applying predetermined energy.

The portable cancer diagnosis device using a thermal analysis method may further include a display unit 70 which displays the determination about the presence of cancer. For the attachable portable cancer diagnosis device which is used being attached to an electronic device, the result may be displayed by the electronic device without an additional display unit.

The portable cancer diagnosis device using a thermal analysis method may further include a power supply unit which supplies power to the heating unit 20. When the power supply unit is equipped additionally, although the size and weight of the device may be increased as compared to the device which receives power supply from the electronic device, it is advantageous in that more elaborate temperature control is possible.

The type of cancer may be one or more type of cancer selected from a group consisting of blood cancer, liver cancer, lung cancer, bladder cancer, stomach cancer, colorectal cancer, bile duct cancer, breast cancer, uterine cancer, colon cancer, ovarian cancer, pancreatic cancer, prostate cancer, bone cancer, skin cancer, laryngeal cancer, nasopharyngeal cancer, small intestine cancer, thyroid cancer, parathyroid cancer, urethral cancer, bronchial cancer, kidney cancer and bone marrow cancer.

The electronic device 80 may be any one selected from a mobile phone, a smartphone, a personal computer, a laptop computer and a tablet PC. The cancer diagnosis information stored in an application or a program may be used for health care of individuals via wireless networks and may be utilized as big data.

Hereinafter, the present disclosure is described in more detail through a test example.

### Test Example 1. Differential scanning calorimetry of blood cancer cells

### Cells

Rat kidney epithelial cells and human promyelocytic leukemia cells HL-60 were purchased from Korean Cell Line Bank.

### Differential scanning calorimetry

Differential scanning calorimetry was conducted with DSC250 (TA Instruments). After maintaining the temperature of a sample at 25 °C for 10 minutes while supplying nitrogen from outside at a rate of 50 mL/min, the temperature was raised to 50 °C at a rate of 2 °C /min.

### Analysis of result

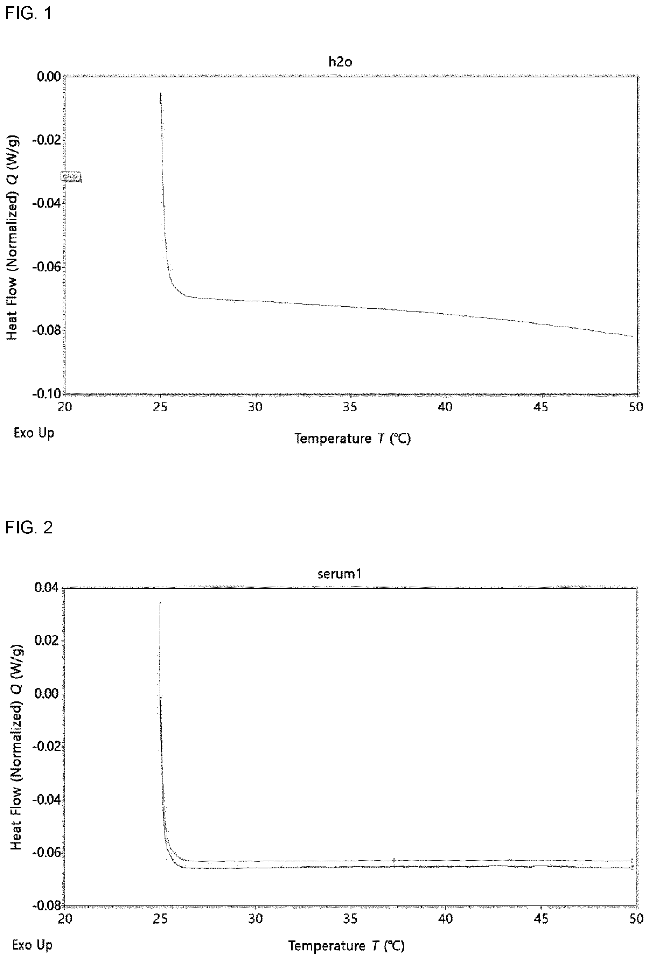

FIG. 1 shows a result of conducting differential scanning calorimetry for water according to an exemplary embodiment of the present disclosure. As can be seen from FIG. 1, there was no peculiar change in the measurement temperature range, suggesting that the differential scanning calorimetry was conducted normally.

Next, differential scanning calorimetry was conducted twice using a medium. FIG. 2 shows a result of conducting differential scanning calorimetry for a medium according to an exemplary embodiment of the present disclosure. As can be seen from FIG. 2, no peculiar change was observed during the differential scanning calorimetry of the medium.

In addition, differential scanning calorimetry was conducted 6 times for the normal cells. FIG. 3A and FIG. 3B show a result of differential scanning calorimetry for the normal cells according to an exemplary embodiment of the present disclosure. As can be seen from FIG. 3A and FIG. 3B, no peculiar change was observed during the normal cells.

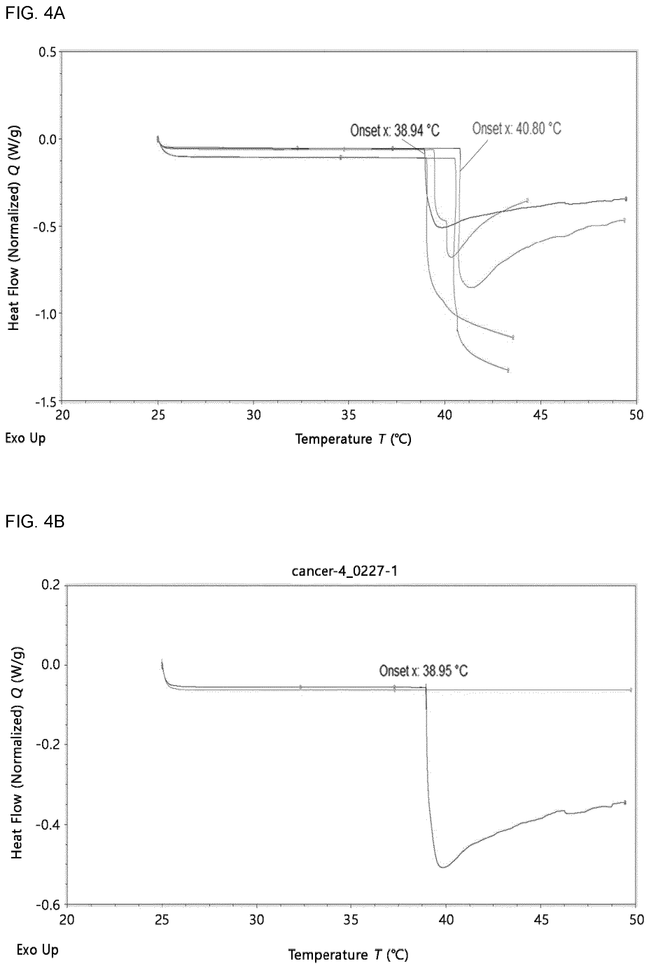

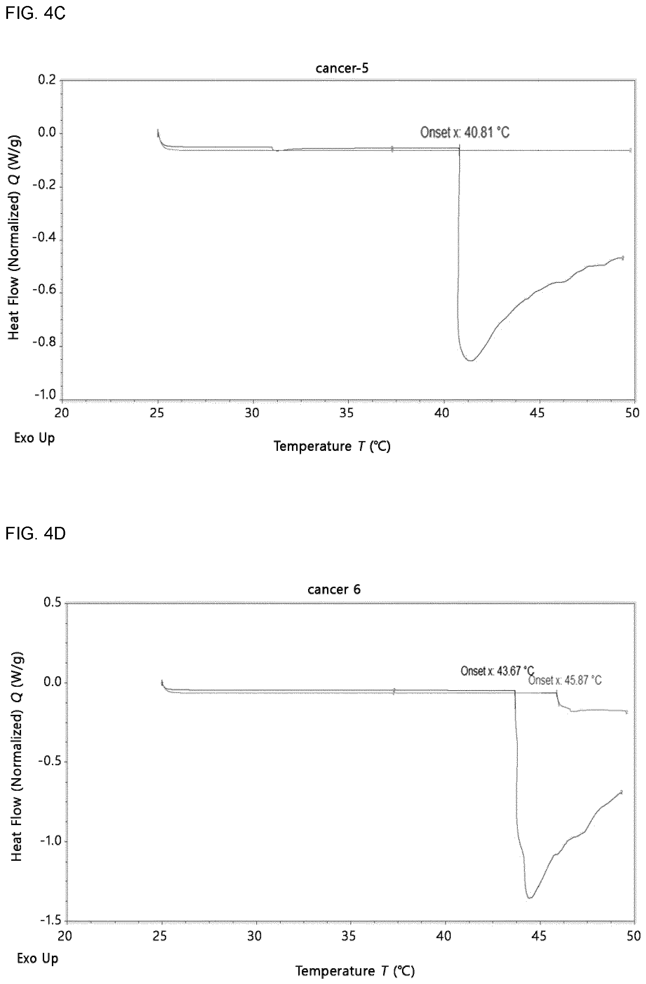

Differential scanning calorimetry was conducted 5 times for the cancer cells. FIG. 4A shows a result of differential scanning calorimetry for a mixture of the cancer cells and a medium according to an exemplary embodiment of the present disclosure, and FIGS. 4B and 4C compare results of differential scanning calorimetry for the normal cells and the cancer cells according to an exemplary embodiment of the present disclosure. FIG. 4D shows a result of additional test for the cancer cells according to an exemplary embodiment of the present disclosure (peculiar result may be observed outside the optimum temperature range).

As can be seen from FIG. 4A, endothermal reaction (including glass transition/phase transition) began at a range of 38.94-40.80 °C during the differential scanning calorimetry of the mixture of the cancer cells and the medium. Also, as can be seen from the comparison of the normal cells and the cancer cells in FIGS. 4B and 4C, the thermochemical reaction was more distinct in the cancer cells. In addition, as can be seen from FIG. 4D, temperature shift occurred depending on the structure, etc. of the cancer cells.

Additionally, differential scanning calorimetry was conducted repeatedly 3 times for the same cancer cells. FIG. 5 shows a result of repeating differential scanning calorimetry 3 times for the same cancer cells according to an exemplary embodiment of the present disclosure. As can be seen from FIG. 5, endothermal reaction began at 39.47 °C during the first differential scanning calorimetry, but peculiar change (heat absorption or transition) could not be observed for the same sample during the second and third analysis due to denaturation or death of the cancer cells.

Accordingly, through the differential scanning calorimetry of blood cancer cells, it was confirmed that thermal reaction occurs for cancer cells in a temperature range of 37-47 °C, unlike normal cells, and cancer diagnosis information such as the presence of cancer can be provided based thereon.

The exemplary embodiments described above are only for illustrating the present disclosure and the present disclosure is not limited thereto. Those having ordinary knowledge in the art to which the present disclosure will be able to carry out the present disclosure by making various changes thereto. Therefore, the scope of technical protection of the present disclosure should be defined by the attached claims.

## Claims

1. A method for providing cancer diagnosis information, comprising:
a step of obtaining a biological sample;
a step of acquiring heat flux data of the biological sample in a temperature range of 37-47 °C; and
a step of providing cancer diagnosis information based on the heat flux data,
wherein the step of providing cancer diagnosis information determines a presence of cancer when the heat flux data shows an onset of an endothermal reaction in a temperature range of 38-45 °C, and
wherein the biological sample is one or more selected from a group consisting of cellular tissue, whole blood, urine, stool, sputum, saliva, tissue, cell and in-vitro cell culture.

2. The method for providing cancer diagnosis information according to claim 1, wherein the heat flux data is one or more selected from a group consisting of thermochemical reaction onset temperature, heat flow change, calorie change, power compensation and compensation temperature.

3. The method for providing cancer diagnosis information according to claim 1, wherein the step of acquiring the heat flux data is performed by differential scanning calorimetry (DSC), isothermal titration calorimetry (ITC) or differential thermal analysis (DTA).

4. The method for providing cancer diagnosis information according to claim 1, wherein the step of acquiring the heat flux data is performed by differential scanning calorimetry and comprises a step of heating the biological sample to 37-47 °C at a rate of 1-10 °C/min.

5. The method for providing cancer diagnosis information according to claim 1, wherein the step of acquiring the heat flux data further comprises a stabilization step maintaining the temperature of the biological sample at 25-30 °C for 1-60 minutes before reaching the temperature at which the heat flux data is acquired.

6. The method for providing cancer diagnosis information according to claim 1, wherein the step of acquiring the heat flux data is performed by differential scanning calorimetry, and, in the step of providing cancer diagnosis information based on the heat flux data, diagnostic information about the presence of cancer is provided based on the thermochemical reaction onset temperature of the biological sample.

7. A portable cancer diagnosis device using a thermal analysis method, comprising:
a sample reception unit to which a biological sample is introduced;
a heating unit which heats the sample reception unit;
a first temperature sensor which measures the temperature of the sample reception unit;
a power supply unit which supplies power to the heating unit; and
a control unit which determines the presence of cancer based on the temperature change data of the sample reception unit with time measured by the first temperature sensor,
wherein the biological sample is one or more selected from a group consisting of cellular tissue, blood and body fluid,
wherein the control unit splits the temperature of the temperature change data of the sample reception unit with time into a time zone A below a first predetermined temperature, a time zone B between the first predetermined temperature and a second predetermined temperature, and a time zone C above the second predetermined temperature, and determines the presence of cancer by measuring elongated time in the time zone B,
wherein the first predetermined temperature is 34-38 °C, and wherein the second predetermined temperature is 42-47 °C.

8. The portable cancer diagnosis device using a thermal analysis method according to claim 7,
wherein the portable cancer diagnosis device using a thermal analysis method further comprises an interface unit which transmits the temperature change data of the sample reception unit with time measured by the first temperature sensor to the control unit of an electronic device and supplies power from the electronic device to the heating unit,
wherein the control unit of the electronic device determines the presence of cancer based on the temperature change data with time transmitted from the interface unit.

9. The portable cancer diagnosis device using a thermal analysis method according to claim 7, wherein the control unit determines the presence of cancer by measuring the temperature of the sample reception unit for a predetermined time from the temperature change data of the sample reception unit with time.

10. The portable cancer diagnosis device using a thermal analysis method according to claim 7, wherein the control unit represents the temperature change data of the sample reception unit with time on a graph with time on the x-axis and temperature on the y-axis, and determines the presence of cancer by analyzing the rough shape of a graph obtained by differentiating the graph with respect to time.

11. The portable cancer diagnosis device using a thermal analysis method according to claim 7, wherein the control unit represents the temperature change data of the sample reception unit with time on a graph with time on the x-axis and temperature on the y-axis, splits the time zone in the graph into a time zone A corresponding to below a first predetermined temperature, a time zone B corresponding to between the first predetermined temperature and a second predetermined temperature, and a time zone C corresponding to above the second predetermined temperature, and determines the presence of cancer by analyzing the rough shape of a graph obtained by differentiating the graph with respect to time for the time zone B.

12. The portable cancer diagnosis device using a thermal analysis method according to claim 7, wherein the portable cancer diagnosis device using a thermal analysis method controls the temperature of the heating unit by applying a fixed power corresponding to a predetermined maximum target temperature to the heating unit.

13. The portable cancer diagnosis device using a thermal analysis method according to claim 7, wherein the portable cancer diagnosis device using a thermal analysis method controls the heating unit to be heated at a predetermined heating rate by raising the power suppled to the heating unit at a predetermined ratio with time.

14. The portable cancer diagnosis device using a thermal analysis method according to claim 7, wherein the portable cancer diagnosis device using a thermal analysis method further comprises a second temperature sensor which measures the temperature of the heating unit, and the control unit controls the heating unit to be heated at a predetermined heating rate using temperature data measured by the second temperature sensor.

15. The portable cancer diagnosis device using a thermal analysis method according to claim 14, wherein the control unit determines the presence of cancer by measuring the power supplied to the heating unit with time.

## Patentansprüche

1. Verfahren zur Bereitstellung von Krebsdiagnoseinformationen,
umfassend:
einen Schritt des Erhaltens einer biologischen Probe;
einen Schritt des Erfassens von Wärmestromdaten der biologischen Probe in einem Temperaturbereich von 37 bis 47 °C; und
einen Schritt des Bereitstellens von Krebsdiagnoseinformationen basierend auf den Wärmestromdaten, wobei der Schritt des Bereitstellens von Krebsdiagnoseinformationen Vorliegen von Krebs bestimmt, wenn die Wärmestromdaten ein Einsetzen einer endothermen Reaktion in einem Temperaturbereich von 38 bis 45 °C zeigen, und
wobei die biologische Probe eine oder mehrere ist, ausgewählt aus einer Gruppe bestehend aus Zellgewebe, Vollblut, Urin, Stuhl, Sputum, Speichel, Gewebe, Zellen und In-vitro-Zellkulturen.

2. Verfahren zur Bereitstellung von Krebsdiagnoseinformationen nach Anspruch 1, wobei die Wärmestromdaten eine oder mehrere Angaben sind, ausgewählt aus einer Gruppe bestehend aus der Temperatur des Beginns einer thermochemischen Reaktion, der Wärmestromänderung, der Kalorienzahländerung, der Leistungskompensation und der Kompensationstemperatur.

3. Verfahren zur Bereitstellung von Krebsdiagnoseinformationen nach Anspruch 1, wobei der Schritt des Erfassens der Wärmestromdaten durch Differentialscanningkalorimetrie (*differential scanning calorimetry* - DSC), isothermische Titrationskalorimetrie (*isothermal titration calorimetry -* ITC) oder Differentialthermoanalyse (*differential thermal analysis* - DTA) durchgeführt wird.

4. Verfahren zur Bereitstellung von Krebsdiagnoseinformationen nach Anspruch 1, wobei der Schritt des Erfassens der Wärmestromdaten mittels Differentialscanningkalorimetrie durchgeführt wird und einen Schritt des Erhitzens der biologischen Probe auf 37 bis 47 °C mit einer Rate von 1 bis 10 °C/min umfasst.

5. Verfahren zur Bereitstellung von Krebsdiagnoseinformationen nach Anspruch 1, wobei der Schritt des Erfassens der Wärmestromdaten ferner einen Stabilisierungsschritt umfasst, bei dem die Temperatur der biologischen Probe 1 bis 60 Minuten lang auf 25-30 °C gehalten wird, bevor die Temperatur erreicht wird, bei der die Wärmestromdaten erfasst werden.

6. Verfahren zur Bereitstellung von Krebsdiagnoseinformationen nach Anspruch 1, wobei der Schritt des Erfassens der Wärmestromdaten mittels Differentialscanningkalorimetrie durchgeführt wird und in dem Schritt des Bereitstellens von Krebsdiagnoseinformationen basierend auf den Wärmestromdaten Diagnoseinformationen über das Vorliegen von Krebs basierend auf der thermochemischen Reaktionseintrittstemperatur der biologischen Probe bereitgestellt werden.

7. Tragbare Krebsdiagnosevorrichtung, die ein Thermoanalyseverfahren verwendet, umfassend:
eine Probenaufnahmeeinheit, in die eine biologische Probe eingeführt wird;
eine Heizeinheit, welche die Probenaufnahmeeinheit erhitzt;
einen ersten Temperatursensor, der die Temperatur der Probenaufnahmeeinheit misst;
eine Stromversorgungseinheit zur Versorgung der Heizeinheit mit Strom; und
eine Steuereinheit, die das Vorliegen von Krebs basierend auf den von dem ersten Temperatursensor gemessenen Temperaturänderungsdaten der Probenaufnahmeeinheit im Laufe der Zeit bestimmt,
wobei die biologische Probe eine oder mehrere ist ausgewählt aus der Gruppe bestehend aus zellulärem Gewebe, Blut und Körperflüssigkeit,
wobei die Steuereinheit die Temperatur der Temperaturänderungsdaten der Probenaufnahmeeinheit im Laufe der Zeit in eine Zeitzone A unterhalb einer ersten vorbestimmten Temperatur, eine Zeitzone B zwischen der ersten vorbestimmten Temperatur und einer zweiten vorbestimmten Temperatur und eine Zeitzone C oberhalb der zweiten vorbestimmten Temperatur aufteilt und das Vorliegen von Krebs durch Messung der verlängerten Zeit in der Zeitzone B bestimmt,
wobei die erste vorbestimmte Temperatur 34 bis 38 °C ist und die zweite vorbestimmte Temperatur 42 bis 47 °C ist.

8. Tragbare Krebsdiagnosevorrichtung, die ein Thermoanalyseverfahren nach Anspruch 7 verwendet,
wobei die tragbare Krebsdiagnosevorrichtung, die ein Thermoanalyseverfahren verwendet, ferner eine Schnittstelleneinheit umfasst, welche die von dem ersten Temperatursensor gemessenen Temperaturänderungsdaten der Probenaufnahmeeinheit im Laufe der Zeit an die Steuereinheit einer elektronischen Vorrichtung überträgt und die Heizeinheit mit Strom von der elektronischen Vorrichtung versorgt,
wobei die Steuereinheit der elektronischen Vorrichtung das Vorliegen von Krebs basierend auf den von der Schnittstelleneinheit übertragenen Temperaturänderungsdaten im Laufe der Zeit bestimmt.

9. Tragbare Krebsdiagnosevorrichtung, die ein Thermoanalyseverfahren nach Anspruch 7 verwendet, wobei die Steuereinheit das Vorliegen von Krebs bestimmt, durch Messen der Temperatur der Probenaufnahmeeinheit für eine vorbestimmte Zeit aus den Temperaturänderungsdaten der Probenaufnahmeeinheit im Laufe der Zeit.

10. Tragbare Krebsdiagnosevorrichtung, die ein Thermoanalyseverfahren nach Anspruch 7 verwendet, wobei die Steuereinheit die Temperaturänderungsdaten der Probenaufnahmeeinheit im Laufe der Zeit auf einem Graphen mit der Zeit auf der x-Achse und der Temperatur auf der y-Achse darstellt und das Vorliegen von Krebs durch Analysieren der groben Form eines Graphen bestimmt, erhältlich durch Differenzieren des Graphen in Bezug auf die Zeit.

11. Tragbare Krebsdiagnosevorrichtung, die ein Thermoanalyseverfahren nach Anspruch 7 verwendet, wobei die Steuereinheit die Temperaturänderungsdaten der Probenaufnahmeeinheit im Laufe der Zeit in einem Graphen mit der Zeit auf der x-Achse und der Temperatur auf der y-Achse darstellt, die Zeitzone in dem Graphen in eine Zeitzone A, die unterhalb einer ersten vorbestimmten Temperatur liegt, aufteilt, eine Zeitzone B, die zwischen der ersten vorbestimmten Temperatur und einer zweiten vorbestimmten Temperatur liegt, und eine Zeitzone C, die über der zweiten vorbestimmten Temperatur liegt, und das Vorliegen von Krebs durch Analysieren der groben Form eines Graphen bestimmt, erhältlich durch Differenzieren des Graphen in Bezug auf die Zeit für die Zeitzone B.

12. Tragbare Krebsdiagnosevorrichtung, die ein Thermoanalyseverfahren nach Anspruch 7 verwendet, wobei die tragbare Krebsdiagnosevorrichtung, die ein Thermoanalyseverfahren verwendet, die Temperatur der Heizeinheit steuert, durch Anlegen einer festen Leistung, die einer vorbestimmten maximalen Zieltemperatur entspricht, an die Heizeinheit.

13. Tragbare Krebsdiagnosevorrichtung, die ein Thermoanalyseverfahren nach Anspruch 7 verwendet, wobei die tragbare Krebsdiagnosevorrichtung, die ein Thermoanalyseverfahren verwendet, die Heizeinheit steuert, dass sie mit einer vorbestimmten Heizrate erhitzt wird, und zwar durch Erhöhung der der Heizeinheit zugeführten Leistung in einem vorbestimmten Verhältnis zur Zeit.

14. Tragbare Krebsdiagnosevorrichtung, die ein Thermoanalyseverfahren nach Anspruch 7 verwendet, wobei die tragbare Krebsdiagnosevorrichtung, die ein Thermoanalyseverfahren verwendet, ferner einen zweiten Temperatursensor umfasst, der die Temperatur der Heizeinheit misst, und die Steuereinheit die Heizeinheit steuert, dass sie unter Verwendung von Temperaturdaten, die von dem zweiten Temperatursensor gemessen werden, mit einer vorbestimmten Heizrate erhitzt wird.

15. Tragbare Krebsdiagnosevorrichtung, die ein Thermoanalyseverfahren nach Anspruch 14 verwendet, wobei die Steuereinheit das Vorliegen von Krebs durch Messung der der Heizeinheit zugeführten Leistung im Laufe der Zeit bestimmt.

## Revendications

1. Procédé destiné à fournir des informations de diagnostic du cancer, comprenant :
une étape consistant à obtenir un échantillon biologique ;
une étape consistant à acquérir des données de flux de chaleur de l'échantillon biologique dans une plage de température de 37 à 47 °C ; et
une étape consistant à fournir des informations de diagnostic du cancer basées sur les données de flux de chaleur,
dans lequel l'étape de fourniture d'informations de diagnostic du cancer détermine une présence de cancer lorsque les données de flux de chaleur montrent un début de réaction endothermique dans une plage de température de 38 à 45 °C, et
dans lequel l'échantillon biologique est un ou plusieurs élément(s) choisi(s) dans un groupe comprenant le tissu cellulaire, le sang total, l'urine, les fèces, le crachat, la salive, le tissu, la cellule et la culture cellulaire *in vitro.*

2. Procédé destiné à fournir des informations de diagnostic du cancer selon la revendication 1, dans lequel les données de flux de chaleur sont un ou plusieurs élément(s) choisi(s) dans un groupe comprenant la température de début de réaction thermochimique, la variation de flux de chaleur, la variation de calories, la compensation de puissance et la température de compensation.

3. Procédé destiné à fournir des informations de diagnostic du cancer selon la revendication 1, dans lequel l'étape d'acquisition des données de flux de chaleur est réalisée par analyse calorimétrique différentielle (ACD), par calorimétrie à titrage isotherme (ITC) ou par analyse thermique différentielle (ATD).

4. Procédé destiné à fournir des informations de diagnostic du cancer selon la revendication 1, dans lequel l'étape d'acquisition des données de flux de chaleur est réalisée par analyse calorimétrique différentielle et comprend une étape consistant à chauffer l'échantillon biologique à 37-47 °C à une vitesse de 1 à 10 °C/min.

5. Procédé destiné à fournir des informations de diagnostic du cancer selon la revendication 1, dans lequel l'étape d'acquisition des données de flux de chaleur comprend en outre une étape de stabilisation maintenant la température de l'échantillon biologique à 25-30 °C pendant 1 à 60 minutes avant d'atteindre la température à laquelle se fait l'acquisition des données de flux de chaleur.

6. Procédé destiné à fournir des informations de diagnostic du cancer selon la revendication 1, dans lequel l'étape d'acquisition des données de flux de chaleur est réalisée par analyse calorimétrique différentielle, et, dans l'étape de fourniture d'informations de diagnostic du cancer basées sur les données de flux de chaleur, des informations de diagnostic concernant la présence d'un cancer sont fournies d'après la température de début de réaction thermochimique de l'échantillon biologique.

7. Dispositif de diagnostic du cancer portable utilisant un procédé d'analyse thermique, comprenant :
une unité de réception d'échantillon dans laquelle on introduit un échantillon biologique ;
une unité de chauffage qui chauffe l'unité de réception d'échantillon ;
un premier capteur de température qui mesure la température de l'unité de réception d'échantillon ;
une unité d'alimentation électrique qui fournit de l'énergie à l'unité de chauffage ; et
une unité de commande qui détermine la présence d'un cancer d'après les données de variation de température de l'unité de réception d'échantillon dans le temps mesurées par le premier capteur de température,
dans lequel l'échantillon biologique est un ou plusieurs élément(s) choisi(s) dans un groupe comprenant le tissu cellulaire, le sang et un fluide corporel,
dans lequel l'unité de commande divise la température des données de variation de température de l'unité de réception d'échantillon dans le temps en une zone temporelle A sous une première température prédéterminée, une zone temporelle B entre la première température prédéterminée et une deuxième température prédéterminée, et une zone temporelle C au-dessus de la deuxième température prédéterminée, et détermine la présence d'un cancer en mesurant un temps allongé dans la zone temporelle B, dans lequel la première température prédéterminée vaut 34-38 °C, et dans lequel la deuxième température prédéterminée vaut 42-47 °C.

8. Dispositif de diagnostic du cancer portable utilisant un procédé d'analyse thermique selon la revendication 7,
dans lequel le dispositif de diagnostic du cancer portable utilisant un procédé d'analyse thermique comprend en outre une unité d'interface qui transmet les données de variation de température de l'unité de réception d'échantillon dans le temps mesurées par le premier capteur de température à l'unité de commande d'un dispositif électronique et fournit de l'énergie du dispositif électronique à l'unité de chauffage,
dans lequel l'unité de commande du dispositif électronique détermine la présence d'un cancer d'après les données de variation de température dans le temps transmises par l'unité d'interface.

9. Dispositif de diagnostic du cancer portable utilisant un procédé d'analyse thermique selon la revendication 7, dans lequel l'unité de commande détermine la présence d'un cancer en mesurant la température de l'unité de réception d'échantillon pendant une durée prédéterminée à partir des données de variation de température de l'unité de réception d'échantillon dans le temps.

10. Dispositif de diagnostic du cancer portable utilisant un procédé d'analyse thermique selon la revendication 7, dans lequel l'unité de commande représente les données de variation de température de l'unité de réception d'échantillon dans le temps sur une courbe avec le temps sur l'axe des x et la température sur l'axe des y, et détermine la présence d'un cancer en analysant la forme globale d'une courbe obtenue en calculant la dérivée de la courbe par rapport au temps.

11. Dispositif de diagnostic du cancer portable utilisant un procédé d'analyse thermique selon la revendication 7, dans lequel l'unité de commande représente les données de variation de température de l'unité de réception d'échantillon dans le temps sur une courbe avec le temps sur l'axe des x et la température sur l'axe des y, divise la zone temporelle de la courbe en une zone temporelle A correspondant à une région sous une première température prédéterminée, une zone temporelle B correspondant à une région entre la première température prédéterminée et une deuxième température prédéterminée, et une zone temporelle C correspondant à une région au-dessus de la deuxième température prédéterminée, et détermine la présence d'un cancer en analysant la forme globale d'une courbe obtenue en calculant la dérivée de la courbe par rapport au temps pour la zone temporelle B.

12. Dispositif de diagnostic du cancer portable utilisant un procédé d'analyse thermique selon la revendication 7, dans lequel le dispositif de diagnostic du cancer portable utilisant un procédé d'analyse thermique règle la température de l'unité de chauffage en appliquant une puissance fixe correspondant à une température cible maximale prédéterminée à l'unité de chauffage.

13. Dispositif de diagnostic du cancer portable utilisant un procédé d'analyse thermique selon la revendication 7, dans lequel le dispositif de diagnostic du cancer portable utilisant un procédé d'analyse thermique commande l'unité de chauffage à chauffer avec une vitesse de chauffage prédéterminée en augmentant la puissance fournie à l'unité de chauffage avec un rapport prédéterminé par rapport au temps.

14. Dispositif de diagnostic du cancer portable utilisant un procédé d'analyse thermique selon la revendication 7, dans lequel le dispositif de diagnostic du cancer portable utilisant un procédé d'analyse thermique comprend en outre un deuxième capteur de température qui mesure la température de l'unité de chauffage, et l'unité de commande commande l'unité de chauffage à chauffer avec une vitesse de chauffage prédéterminée en utilisant des données de température mesurées par le deuxième capteur de température.

15. Dispositif de diagnostic du cancer portable utilisant un procédé d'analyse thermique selon la revendication **14,** dans lequel l'unité de commande détermine la présence d'un cancer en mesurant l'énergie fournie à l'unité de chauffage dans le temps.
